# EUROPÄISCHE PATENTANMELDUNG

(11) **EP 4 846 984 A1**
(43) Veröffentlichungstag der Anmeldung: **02.12.2026**
(21) Anmeldenummer: 25178853.5
(22) Anmeldetag: 26.05.2025
(51) Int. Cl.: A61F 2/78, A61F 2/50

(54) **VERFAHREN ZUR HERSTELLUNG EINES SILIKONKÖRPERS, SILIKONKÖRPER, PROTHESE UND ÜBERZUG FÜR EINE PROTHESE**

(71) Anmelder: SiliCore GmbH, 8306 Brüttisellen (CH)
(72) Erfinder: MARKWALDER, Martin, 8306 Brüttisellen (CH); VON ESSEN, Stefan, 14943 Luckenwalde (CH)
(74) Vertreter: Gulde & Partner

(57) **Zusammenfassung**

Die Erfindung betrifft ein Verfahren zur Herstellung eines Silikonkörpers (10) und einen Silikonkörper (10). Ferner umfasst die Erfindung eine Prothese (100) und einen Überzug für eine Prothese (100).

Es ist vorgesehen, dass der gelblich blasse Grundfarbton von Haut, der durch eine Blanchierung der Haut unter Krafteinwirkung auftritt, individuell für den Patienten (12) erfasst und zur Einfärbung des Silikons für einen Silikonkörper (10) verwendet wird. Durch die so erreichte Farbgebung des Silikonkörpers (10) lässt sich die individuelle Naturfarbe des Patienten (12) auf einfache Weise und möglichst originalgetreu wiedergeben.

## Beschreibung

Die Erfindung betrifft ein Verfahren zur Herstellung eines Silikonkörpers und einen Silikonkörper. Ferner betrifft die Erfindung eine Prothese und einen Überzug für eine Prothese.

Wenn ein Mensch mit einer Amputation versorgt wird, hängt diese von den entsprechenden Umständen und Bedürfnissen der Person ab. Um fehlende oder eingeschränkte Körperteile zu ersetzen oder zu vervollständigen werden bekanntermaßen Prothesen eingesetzt. Zur Herstellung einer Prothese wird dabei beispielsweise zunächst ein grober Prothesenrohling erstellt, der durch einen Orthopädietechniker an die Form und Gestalt des betroffenen Körperteils sukzessive und individuell angepasst wird. Der Patient begibt sich häufig mehrmals zum Orthopädietechniker, um die individuell angefertigte Prothese abzustimmen. Insbesondere um lebensechte Prothesen herzustellen, müssen feine Oberflächenstrukturen händisch in die individuelle Prothese eingebracht und die Farbgebung der Prothese aufwändig mittels Airbrush oder Pinsel an den Patienten angepasst werden.

Wenn also eine lebensechte, individuelle Kosmetik gewünscht ist, egal ob als kosmetischer Überzug für eine Prothese, beispielsweise für eine Hand- oder Armprothese oder als Habitusprothese, ist eine originalgetreue Farbwiedergabe der Haut des Patienten wünschenswert.

Der Arbeitsprozess zum Erreichen einer originalgetreuen farblichen Gestaltung einer Prothese oder eines Prothesenüberzugs ist äußerst schwierig und stellt enorme Limitierungen dar: Die farbliche Gestaltung erfordert meist das "Einkneten" unterschiedlich eingefärbter Silikonteile, die künstlerisch-handwerkliche Fähigkeit des Technikers ist maßgeblich entscheidend über den lebensechten Charakter der Prothese oder des Prothesenüberzugs und es wird durch diesen Arbeitsprozess immer nur ein Einzelstück gefertigt.

Daher wäre es wünschenswert, die Herstellung von originalgetreuen Prothesen aus Silikonen zu vereinfachen.

Der Erfindung liegt daher nun die Aufgabe zugrunde, eine Möglichkeit zu schaffen, lebensechte Prothesen aus Silikon kostengünstig herzustellen.

Die erfindungsgemäße Aufgabe wird gelöst durch ein Verfahren zur Herstellung eines Silikonkörpers, einen Silikonkörper, eine Prothese und einen Überzug für eine Prothese gemäß den unabhängigen Patentansprüchen. Bevorzugte Weiterbildungen sind Gegenstand der jeweils rückbezogenen Unteransprüche.

Ein Aspekt betrifft ein Verfahren eines Silikonkörpers, insbesondere für eine Prothese oder für einen Überzug für eine Prothese. Gemäß der vorliegenden Offenbarung umfasst der Begriff Prothese bevorzugt die vollständige Prothese oder nur eines Teiles davon und der Begriff Überzug umfasst bevorzugt den vollständigen Überzug oder nur einen Teil davon. Mit anderen Worten ist das Verfahren bevorzugt ein Verfahren zur Herstellung eines Silikonkörpers für eine Prothese oder eines Teiles davon bzw. für einen Überzug für eine Prothese oder einen Teil davon. Die hergestellte Prothese beziehungsweise der Überzug dafür dienen insbesondere zum vollständigen oder teilweisen Ersatz einer Extremität, wie beispielsweise eines Fingers, einer Hand, eines Arms, eines Beins, eines Fußes, eines Ohres und dergleichen.

Gemäß dem Verfahren wird ein Hautbereich eines Patienten mit Kraft zum Blanchieren der Haut beaufschlagt. Mit anderen Worten wird in dem Hautbereich des Patienten durch eine Krafteinwirkung der sogenannte Blanching-Effekt (französisch "blanc" - deutsch "weiß") hervorgerufen. Der Blanching-Effekt tritt auf, wenn auf eine Stelle der Haut eine Kraft (oder ein Druck) ausgeübt wird, und das Blut aus den oberflächlichen Kapillaren verdrängt wird. Dadurch erscheint die Haut an dieser Stelle weiß gelblich bis elfenbeinfarben. Sobald die Kraft (oder der Druck) nachlässt, kehrt die normale (also unblanchierte) Hautfarbe zurück, da das Blut wieder einströmt. Bei einem gesunden Körperteil ist der Blanching-Effekt reversibel, d.h. dass sich die Haut nach kurzer Zeit wieder zurück färbt, sobald der Druck nachlässt und die Durchblutung zurückkehrt. Daher wird dieser Effekt beispielsweise in der Medizin zur Beurteilung von Durchblutung und Hautveränderungen genutzt. Erfindungsgemäß, hingegen, wird dieser Effekt ausgenutzt, um die Grundtonfarbe eines Silikonkörpers oder eines Rohlings gemäß dem Farbton von blanchierter Haut festzulegen.

In einem weiteren Verfahrensschritt wird ein Farbton des blanchierten Hautbereichs erfasst. Bevorzugt wird der erfasste Farbton des blanchierten Hautbereichs in einer numerischen Form beschrieben. Numerische Formen können von einem elektronischen Gerät, wie einem Computer, einem Display oder grundsätzlich mittels Software korrekt interpretiert werden. Beispiele für numerische Farbsysteme sind das RGB-Farbmodell (Red, Green, Blue), der HEX-Code (Hexadezimaldarstellung des RGB-Wertes), das HSL-Farbmodell (Hue, Saturation, Lightness) und der Lab-Farbraum (CIELAB). Besonders bevorzugt wird der Lab-Farbraum verwendet, also L*: Helligkeit (0 = Schwarz, 100 = Weiß), a*: grün (-) bis rot (+) und b*: blau (-) bis gelb (+), da dieser nah farbwissenschaftlich genauer ist und näher an der menschlichen Wahrnehmung liegt. Mit anderen Worten wird bevorzugt ein Lab-Wert des blanchierten Hautbereichs erfasst.

Ferner wird ein Silikon gemäß dem erfassten Farbton des blanchierten Hautbereichs eingefärbt. Die Einfärbung von Silikon gemäß einem vorgegebenen Farbton ist dem Fachmann grundsätzlich bekannt. Beispielsweise können Farbpigmente in das Silikon eingeknetet oder in eine Silikon-haltige Lösung eingemischt werden. Das Silikon ist hierzu bevorzugt noch nicht ausvulkanisiert. Mit anderen Worten das einzufärbende Silikon in einem unvulkanisierten Zustand vor.

In einem weiteren Schritt des erfindungsgemäßen Verfahrens wird ein Silikonkörper basierend auf dem eingefärbten Silikon hergestellt. Mit anderen Worten wird das eingefärbte Silikon in eine gewünschte Form gebracht, also ausgeformt, und getempert. Bevorzugt wird der ausgeformte Silikonkörper entsprechend den Herstellerangaben des gewählten Silikons in einem Ofen getempert, um den Vulkanisierungsprozess des Silikons zu bewirken. Beispielsweise wird der Silikonkörper bei 90 °C für zwei Stunden getempert. Durch den Vulkanisierungsprozess erhält das Silikon die zur späteren Verwendung gewünschten mechanischen Eigenschaften.

Der Erfindung liegt die Einsicht zugrunde, dass der Farbton der blanchierten Haut entscheidend ist, um die individuelle Naturfarbe der Haut eines Patienten originalgetreu in einem Silikonkörper, beispielsweise einer Prothese oder einem Prothesenüberzug, wiedergeben zu können und damit eine besonders lebensechte Prothese oder Prothesenüberzug zu erreichen. Es wurde erkannt, dass sich der Farbton der blanchierten Haut von Patient zu Patient (zumindest in Nuancen) unterscheidet. Die oberste Hautschicht, das sogenannte Stratum corneum, besteht aus verhornten Hautzellen, die das Protein Keratin enthalten, welches gelb-weißlich ist und vermutlich zum Grundfarbton der blanchierten Haut beiträgt. Ferner ist subkutanes Fettgewebe, welches unter der Haut liegt, gelblich gefärbt, welches bei dem fehlenden "Rotfilter" aufgrund des verdrängten Blutes durchzuschimmern scheint. Unterschiede in den Grundfarbtönen von blanchierter Haut zwischen den Patienten könnten neben der genetischen Herkunft vermutlich auch dadurch bedingt sein, dass das Fettgewebe gelb- bis orangefarbene Carotinoide speichert, vor allem Beta-Carotin, welches mit der Nahrung, beispielweise aus Karotten, Süßkartoffeln, Kürbissen und dergleichen, aufgenommen wird, und die Anzahl von eingespeicherten Carotinoiden entsprechend der Essgewohnheiten des jeweiligen Patienten unterschiedlich ist.

Jedenfalls erschwert die pauschale Verwendung eines einheitlich gefärbten Silikonkörperrohlings für alle Patienten - meist in der unblanchierten "Hautfarbe", wie es gängige Praxis ist - die originalgetreue Farbwiedergabe erheblich. Bei einem falsch gewählten Grundfarbton des Silikonkörperrohlings ist es nahezu unmöglich oder zumindest nur unter enormen Aufwand möglich, die originalgetreue Hautfarbe des Patienten in der Prothese oder dem Prothesenüberzug, beispielsweise durch Auftragen von weiteren Farbschichten auf den Rohling, wiedergeben zu können. Überraschenderweise wird die originalgetreue Farbwiedergabe der Haut des Patienten bei der Prothese oder des Prothesenüberzugs vergleichsweise einfach durch die Berücksichtigung des individuellen Farbtons der blanchierten Haut des Patienten bei der Farbgebung des Silikonkörperrohlings für die (spätere) Prothese oder den Prothesenüberzug des Patienten ermöglicht. Folglich wird erfindungsgemäß nicht nur der Aufwand für die originalgetreue Farbwiedergabe reduziert, sondern auch die Übereinstimmung der Farben grundsätzlich verbessert, wodurch die Lebensechtheit der (späteren) Prothese oder des Prothesenüberzugs verbessert wird.

In bevorzugter Ausgestaltung ist vorgesehen, dass die beaufschlagte Kraft mindestens 0,8 N oder mindestens 1 N beträgt. Die Kraft wird bevorzugt orthogonal zur Haut beaufschlagt, ohne hierauf beschränkt zu sein. Versuche haben gezeigt, dass - je nach Beschaffenheit der Haut des Patienten, wie Hautdicke, Hautalter, betrachtetes Körperteil und dergleichen - bereits ab 0,8 N erste Farbtonveränderungen durch den Blanching-Effekt zu verzeichnen sind. Durch die Beaufschlagung einer vergleichsweisen geringen Kraft ist es besonders einfach, die erfindungsgemäßen Vorteile, insbesondere einen hinsichtlich der Lebensechtheit verbesserten Silikonkörper, zu verwirklichen. Bevorzugt beträgt die beaufschlagte Kraft mindestens 1,5 N, mindestens 2 N, mindestens 2,5 N oder mindestens 3 N.

In weiterer bevorzugter Ausgestaltung ist vorgesehen, dass die beaufschlagte Kraft in einem Kraftbereich liegt, in welchem eine weitere Erhöhung der beaufschlagten Kraft keine weitere Farbtonveränderung der blanchierten Haut erzeugt. Versuche haben gezeigt, dass es eine Kraftschwelle gibt, bei deren Überschreitung jede weitere Erhöhung der Kraft keine weitere Farbtonveränderungen der Haut durch den Blanching-Effekt bewirkt. Mit anderen Worten gibt es einen Kraftbereich, in welchem der Farbton des blanchierten Hautbereichs unverändert bleibt. Die Kraftschwelle hängt von der Beschaffenheit der Haut, insbesondere einer Hautdicke und dem betrachteten Körperteil, ab, und kann zwischen 10 N und 20 N liegen. Das untere Ende des Kraftbereichs ist durch diese Kraftschwelle gegeben. Das obere Ende des Kraftbereichs bezieht sich auf eine maximale Kraftschwelle, bis zu welcher der Patient beziehungsweise das mit Kraft beaufschlagte Körperteil keinen (dauerhaften) Schaden im Sinne einer Verletzung erleidet. Bevorzugt beträgt die beaufschlagte Kraft mindestens 10 N, besonders bevorzugt mindestens 20 N. Besonders bevorzugt wird ein Mindestwert der beaufschlagten Kraft abhängig von der Beschaffenheit der Haut, beispielsweise vom jeweiligen Körperteil und der jeweiligen Hautdicke des Patienten, bestimmt. Durch die Beaufschlagung von Kraft oberhalb der Kraftschwelle, beziehungsweise innerhalb des oben genannten Kraftbereichs, wird verlässlich und reproduzierbar sichergestellt, dass der gewünschte Grundfarbton der Haut des Patienten erfasst werden kann. Insofern können Fehler beim Erfassen des Farbton des blanchierten Hautbereichs reduziert werden, wodurch die Herstellung eines lebensechten Silikonkörpers weiter vereinfacht wird.

In weiterer bevorzugter Ausgestaltung ist vorgesehen, dass das Erfassen des Farbtons des blanchierten Hautbereichs unter Verwendung eines Farbmusterrings und/oder eines zum Erfassen eines Farbtons eingerichteten elektronischen Messgeräts erfolgt. Der Farbmusterring kann eine Vielzahl von verschiedenen Farbmustern mit definierten Farbtönen umfassen, welche neben den blanchierten Hautbereich gelegt werden können, um eine Farbübereinstimmung des blanchierten Hautbereichs mit einem der Farbmuster des Farbmusterrings zu ermitteln. Dadurch, dass jedes Farbmuster einen definierten Farbton umfasst, ist das Erfassen des Farbtons des blanchierten Hautbereichs auf diese Weise besonders einfach möglich. Als zum Erfassen eines Farbtons eingerichtetes elektronisches Messgerät wird bevorzugt ein Spektrometer oder ein Chromameter verwendet. Ein Spektrometer erlaubt es, die Intensität von Licht in Abhängigkeit der Wellenlänge zu erfassen. Mit anderen Worten kann mit einem Spektrometer ein Farbspektrum mit physikalischen Parametern erfasst und ausgegeben werden. Ein Chromameter ist dazu eingerichtet, Farbwerte in einem standardisierten Farbraum - typischerweise CIELAB (L*a*b*) - zu messen und diese als numerische Werte auszugeben. Die Verwendung des elektronischen Messgeräts bietet eine objektive, reproduzierbare und einfache Art und Weise, einen Farbton des blanchierten Hautbereichs zu erfassen.

In weiterer bevorzugter Ausgestaltung ist vorgesehen, dass die beaufschlagte Kraft zum Blanchieren der Haut mit dem Farbmusterring und/oder dem elektronischen Messgerät ausgeübt wird. Im Falle eines Farbmusterrings kann dieser beispielsweise auf eine durchsichtige Platte gelegt werden und die Platte auf die Haut gepresst werden, um den Blanching-Effekt hervorzurufen und den Farbton des blanchierten Hautbereichs anhand des Farbmusterrings zu erfassen. Das elektronische Messgerät umfasst bevorzugt einen lichtdurchlässigen (Glas-) Vorsatz zum Abtasten der Haut, der es ermöglicht, die Haut mit Kraft zu beaufschlagen und währenddessen den Farbton des blanchierten Hautbereichs zu messen. Insofern ist eine besonders einfache und schnelle Art und Weise zum Erfassen des Farbtons des blanchierten Hautbereichs gegeben. Bei einem beispielhaften Durchmesser des Aufsatzes des elektronischen Messgeräts von 8 mm ergibt sich bei einer beaufschlagten Kraft von 1 N ein Druck auf die Haut von etwa 19,9 kPa.

In weiterer bevorzugter Ausgestaltung ist vorgesehen, dass der Farbton des blanchierten Hautbereichs unter Verwendung eines Abgleichs mit Farbtönen einer digitalen Farbbibliothek erfasst wird. Die Verwendung einer digitalen Farbbibliothek erlaubt eine computergestützte Erkennung und reproduzierbare Zuordnung des erfassten Farbtons des blanchierten Hautbereichs. Anhand von numerischen Werten, beispielsweise Lab-Werten, ermittelt ein Computerprogramm den zu dem erfassten Farbton des blanchierten Hautbereichs zugehörigen ähnlichsten Farbton aus der digitalen Farbbibliothek. Folglich wird die Herstellung eines lebensechten Silikonkörpers weiter vereinfacht. Insbesondere kann die Farbbibliothek zu den darin enthaltenden Farbtönen ferner direkt zugeordnete Handlungsanweisungen enthalten, wie der jeweilige Farbton auf dem Silikonkörper erzielt werden kann. Dies betrifft insbesondere die dazu erforderlichen Farben, die jeweilige Stärke des Farbauftrags und mögliche Anzahlen von Farbschichten. Bevorzugt wurde die digitale Farbbibliothek unter Verwendung des elektronischen Messgeräts erstellt, welches ebenfalls zum Erfassen des Farbtons des blanchierten Hautbereichs verwendet wird. Hierdurch können gerätespezifische Fehler bei der Farbtonerfassung reduziert werden.

In weiterer bevorzugter Ausgestaltung ist vorgesehen, dass der Farbton des blanchierten Hautbereichs am Handrücken und/oder am Fußrücken des Patienten erfasst wird. Mit anderen Worten wird die Haut an der Rückseite der Hand (dorsal), also auf der Seite, auf der sich die Fingernägel befinden, und/oder die Oberseite des Fußes (dorsal), also die Seite mit den Sehnen, die sichtbar werden, wenn die Zehen gestreckt werden, mit Kraft zum Blanchieren der Haut beaufschlagt und der Farbton des dort blanchierten Hautbereichs erfasst. Da die Dicke der Haut am Handrücken (dorsal) und am Fußrücken (dorsal) dünner ist als auf den jeweils gegenüberliegenden Seiten, also die Handfläche (palmar) oder die Fußsohle (plantar), ist hier jeweils eine geringere Kraft zum Erzeugen des blanchierten Hautbereichs erforderlich, wodurch das Erfassen des Farbtones des blanchierten Hautbereichs einfacher ist. Insbesondere erfordert der Kraftbereich, in welchem eine weitere Erhöhung der beaufschlagten Kraft keine weitere Farbtonveränderung der blanchierten Haut erzeugt, eine vergleichsweise geringere Kraftbeaufschlagung. Durch die geringere Kraftbeaufschlagung ist das Verfahren einfacher umsetzbar.

In weiterer bevorzugter Ausgestaltung ist vorgesehen, dass eine Vielzahl von Hautbereichen des Patienten zum Blanchieren der Haut mit Kraft beaufschlagt wird. Bevorzugt werden die Farbtöne der Vielzahl von blanchierten Hautbereichen erfasst und das Silikon basierend auf den erfassten Farbtönen der Vielzahl von blanchierten Hautbereichen eingefärbt. Bevorzugt wird ein Mittelwert der erfassten Farbtönen der Vielzahl von blanchierten Hautbereichen berechnet und das Einfärben des Silikons erfolgt basierend auf dem berechneten Mittelwert der erfassten Farbtönen der Vielzahl von blanchierten Hautbereichen. Hierdurch können natürliche Schwankungen und daraus resultierende Fehler beim Erfassen des Farbtons des blanchierten Hautbereichs auf einfache Weise reduziert werden. Die Farbtöne der Vielzahl von blanchierten Hautbereichen werden bevorzugt simultan oder zeitlich versetzt erfasst.

In weiterer bevorzugter Ausgestaltung ist vorgesehen, dass das Verfahren ferner ein Auftragen einer oder einer Vielzahl von Farbschichten auf den hergestellten Silikonkörper umfasst. Durch das Auftragen von Farbschichten auf den eingefärbten Silikonkörper kann dieser in lebensechter Farbgestaltung realisiert werden. So wie das für den Blanching Effekt verdrängte Blut als "Rotfilter" dient, dient/en die aufgetragene/n Farbschicht/en ebenfalls als "Rotfilter", um die lebensechte Farbwiedergabe des Silikonkörpers zu erreichen. Der Auftrag von Farbschichten erlaubt, eine Farbgebung in Schichten von verschiedenen Farben, beispielsweise kaltes rot; warmes rot; gelb; blau; violett und dergleichen, aufzubauen, um eine lebensechte Farbwirkung zu erzielen. Der Farbauftrag erfolgt bevorzugt mittels Drucker, Airbrush, Pinsel und dergleichen.

In weiterer bevorzugter Ausgestaltung ist vorgesehen, dass das Verfahren ferner ein Erfassen eines Farbtons des Hautbereichs in unblanchierter Form umfasst, wobei das Auftragen einer oder einer Vielzahl von Farbschichten auf den hergestellten Silikonkörper basierend auf dem erfassten Farbton des Hautbereichs in unblanchierter Form erfolgt. Der Hautbereich in unblanchierter Form entspricht im Wesentlich der normalen Hautfarbe, die ohne (externe) Kraftbeaufschlagung zu vernehmen ist. Mit anderen Worten wird die normale Hautfarbe des Patienten erfasst und der Auftrag der Farbschichten so durchgeführt, dass der hergestellte Silikonkörper die normale Hautfarbe des Patienten aufweist. Hierzu werden bevorzugt kalibrierte Fotos der Hautbereiche des Patienten unter genormten Lichtquellenzufuhr aufgenommen. Der Farbauftrag erfolgt bevorzugt unter den gleichen Lichtbedingungen, also entsprechend der bei der Fotoaufnahme verwendeten genormten Lichtquelle. Hierdurch wird sichergestellt, dass die farbliche Wiedergabe des Silikonkörpers möglich exakt mit der Hautfarbe des Patienten übereinstimmt. Ferner ist die Anwesenheit des Patienten für den Arbeitsschritt des Farbauftrags nicht erforderlich.

In weiterer bevorzugter Ausgestaltung ist vorgesehen, dass der erfasste Farbton des blanchierten Hautbereichs an einen Server mittels eines Kommunikationssignals übermittelt wird und das Einfärben des Silikons basierend auf dem auf dem Server hinterlegten erfassten Farbton des blanchierten Hautbereichs erfolgt. Hierdurch ist es möglich, den Patienten ortsunabhängig mit einem lebensechten individuellen Silikonkörper versorgen zu können. Die Anwesenheit des Patienten für das Herstellen des Silikonkörpers ist hierbei nicht erforderlich. Die Erfassung des Farbtons des blanchierten Hautbereichs kann demnach beispielsweise durch einen Mitarbeiter in einem lokalen Sanitätshaus vor Ort beim Patienten erfasst und auf den Server übertragen werden. Der Hersteller des Silikonkörpers kann demnach ortsunabhängig und auf einfache Weise lebensechte Silikonkörper für jeden Patienten individuell herstellen. Bevorzugt wird der oben genannte Farbtons des Hautbereichs in unblanchierter Form ebenfalls per Kommunikationsignal an Server übertragen und das Auftragen der einen oder der Vielzahl von Farbschichten auf den hergestellten Silikonkörper erfolgt dann basierend auf dem auf dem Server hinterlegten erfassten Farbton des Hautbereichs in unblanchierter Form.

In weiterer bevorzugter Ausgestaltung ist vorgesehen, dass das Verfahren ferner ein Lösen von Silikon in einem Lösungsmittel zum Erzeugen einer Silikon-haltigen Lösung umfasst, wobei die

Silikon-haltige Lösung gemäß dem erfassten Farbton des blanchierten Hautbereichs eingefärbt wird. Bevorzugt umfasst das Herstellen des Silikonkörpers basierend auf dem eingefärbten Silikon ferner die Schritte eines Ausschwenkens einer Negativform einer Prothese oder einer Negativform eines Überzugs oder eines Übergießens und/oder Eintauchens einer Positivform der Prothese oder einer Positivform des Überzugs. Für das Ausschwenken oder das Übergießen und/oder das Eintauchen wird bevorzugt jeweils ein Fluid verwendet, welches die Silikon-haltige Lösung enthält oder daraus besteht. Bevorzugt wird die Silikon-haltige Lösung zur Einfärbung mit Farbpigmenten versehen. Dies ermöglicht eine einfachere Farbgestaltung des Silikons durch Einrühren im Gegensatz zum Einwalzen, wie es bei manchen Silikonen erforderlich ist. Durch das Ausschwenken beziehungsweise Übergießen und/oder Eintauchen einer Form mit der bzw. in die Silikon-haltigen Lösung können Silikonkörper mit lebensechten Strukturen einfach und kostengünstig hergestellt werden. Durch die Verwendung von Negativ- bzw. Positivformen wird für die Herstellung einer weiteren Prothese (also jede weitere Versorgung des Patienten) etwa 70% der Arbeitszeit eingespart. Außerdem ist dieser Prozess zuverlässig und nicht (maßgeblich) abhängig von der Fähigkeit oder der Tagesform der die Prothese herstellenden Person. Bevorzugt wird die Negativform und/oder die Positivform mittels 3D-Druckverfahren hergestellt. Die Negativform und/oder die Positivform werden bevorzugt durch einen SLS- oder Kunstharz-3D-Drucker ausgedruckt. SLS-3D-Drucker (Selective Laser Sintering) zeichnen sich durch eine hohe Auflösung und durch im Wesentlichen nicht (für das menschliche Auge) sichtbare Schichtübergange, d.h. keine einzelnen sichtbaren Schichten, aus. Bei der Verwendung des SLS-3D-Druckers wird eine Schicht feinkörniges Pulver in den Drucker eingegeben und durch einen Laser zu einem Teil verschmolzen. Kunstharz-3D-Drucker zeichnen sich durch ihre besondere Druckqualität aus. Kunstharz-3D-Drucker verwenden 3D-Druckverfahren wie Stereolithografie (SLA) oder digitale Lichtverarbeitung (Digital Light Processing - DLP), wodurch die Auflösung durch den optischen Punkt des Lasers oder des Projektors (SLA) beziehungsweise des Pixels (DLP) bestimmt wird. Da Licht für die Polymerisation verwendet wird, wird beim Drucken keine Kraft angewendet, wodurch besonders glatte Oberflächen erreicht werden.

In weiterer bevorzugter Ausgestaltung ist vorgesehen, dass das Silikon für den Silikonkörper ein HTV-Silikon umfasst oder daraus besteht. Mit anderen Worten ist der hergestellte Silikonkörper ein HTV Silikonkörper. HTV steht für hoch-temperatur vernetzend (High Temperature Vulcanizing - HTV). Die Hoch-Temperatur bezieht sich dabei auf die Vulkanisierungstemperatur der Silikone und liegt bei mindestens 70 °C, bevorzugt bei mindestens 90 °C, besonders bevorzugt bei mindestens 100 °C. HTV Silikone sind bevorzugt Gummi mit hoher Konsistenz (High Consistency Rubber - HCR), wärmegehärtetes Gummi (Heat Cured Rubber) oder HTV Silikonkautschuk. Anders ausgedrückt ist ein HTV Silikon (vor dem Vulkanisierungsprozess) ein knetbares Feststoffsilikon. Bei HTV Silikonen handelt es sich um eine bestimmte Art von Silikon, die sich durch exzellente mechanische Eigenschaften, wie hohe Bruchdehnung und Weiterreißfestigkeit auszeichnet. Etwas, das bei einer täglich getragenen Prothese, die hohen Belastungen ausgesetzt ist unbedingt notwendig ist, um eine adäquate Lebensdauer zu gewährleisten. Es wird vermutet, dass das knetbare (pastöse) HTV Silikon deshalb so gute physikalische Eigenschaften hat, weil hauptsächlich Kieselsäure als Füllstoff enthalten ist. Die feinen Partikel der Kieselsäure sorgen für eine mechanische Verstärkung des Silikons, indem sie die Polymerketten stützen. Dies führt dazu, dass der Kautschuk eine feste, aber formbare (knetbare) Konsistenz aufweist. Entgegen anderer Silikone, wie kaltvernetzende (Raumtemperatur vernetzend - RTV) Silikone oder Flüssig-Silikone (*Liquid Silicone Rubber -* LSR), musste HTV Silikon bisher als Feststoff verarbeitet werden. Es wurde jedoch ebenfalls herausgefunden, dass HTV Silikon in einem Lösungsmittel gelöst werden kann. Mit anderen Worten wird ein HTV-Silikon, also ein Festsilikonkautschuk, welches normalerweise als Festkörper verarbeitet wird, verdünnt, also in einen flüssigem bzw. fließfähigem Zustand überführt, um anschließend als Fluid oder Lösung in flüssigem bzw. fließfähigem Zustand verarbeitet werden zu können. Es wurde festgestellt, dass die Verarbeitung von HTV Silikon als HTV Silikon-haltige Lösung eine vereinfachte Verarbeitung des HTV Silikons ermöglicht, ohne die (gewünschten) mechanischen Eigenschaften des HTV Silikons im vulkanisierten Endprodukt zu verändern bzw. zu verlieren. Bevorzugt ist das Lösungsmittel ausgewählt aus Hexamethyldisiloxan, Octamethyltrisiloxan, weitere Lösungsmittel der Silangruppe, Kaltreiniger, Heptan, Kohlenwasserstoffe C6-C7, N-Alkane, Iso-Alkane, Cyclische Verbindungen und N-Hexana (z.B. Wasch-Benzin). Insbesondere Hexamethyldisiloxan, Heptan und Wasch-Benzin sind besonders gut geeignete Lösungsmittel für HTV Silikone, die eine besonders gute Löslichkeit des HTV Silikons bieten. Die Wandstärke des hergestellten HTV Silikonkörpers beträgt bevorzugt mindestens 0,15 mm, vorzugsweise mindestens 0,5 mm und/oder höchstens 1,5 mm, vorzugsweise höchstens 1,25 mm, besonders bevorzugt höchstens 1 mm.

Ein weiterer Aspekt betrifft einen Silikonkörper für eine Prothese oder einen Überzug für eine Prothese. Der Silikonkörper ist durch das oben genannte Verfahren zur Herstellung eines Silikonkörpers herstellbar. Die mit den Verfahren beschriebenen optionalen Merkmale und deren Vorteile werden analog in dem Silikonkörper verwirklicht und sind daher beliebig miteinander kombinierbar. Bevorzugt stellt der Silikonkörper eine Silikon-haltige Prothese oder einen Silikon-haltigen Überzug für eine Prothese dar, die durch das oben genannte Verfahren zur Herstellung eines Silikonkörpers herstellbar sind. Der erfindungsgemäß hergestellte Silikonkörper zeichnet sich demnach durch eine Farbgebung entsprechend des erfassten Farbtons der blanchierten Haut aus. Bevorzugt ist der hergestellte Silikonkörper vollständig eingefärbt. Insbesondere bei einem entsprechend ergänzten Farbauftrag auf den Silikonkörper wird die lebensechte Farbwiedergabe der Hautfarbe des Patienten erreicht.

Ein weiterer Aspekt betrifft eine Prothese oder einen Überzug für eine Prothese umfassend den obigen Silikonkörper. Die mit dem Silikonkörper beschriebenen optionalen Merkmale und deren Vorteile werden analog in der Prothese oder dem Überzug verwirklicht und sind daher beliebig miteinander kombinierbar.

Weitere bevorzugte Ausgestaltungen der Erfindung ergeben sich aus den übrigen, in den Unteransprüchen genannten Merkmalen.

Die verschiedenen in dieser Anmeldung genannten Ausführungsformen der Erfindung sind, sofern im Einzelfall nicht anders ausgeführt, mit Vorteil miteinander kombinierbar.

Die Erfindung wird nachfolgend in Ausführungsbeispielen anhand der zugehörigen Zeichnungen erläutert. Es zeigen:
- Figur 1: eine schematische Darstellung eines Verfahrens zum Herstellen eines Silikonkörpers gemäß einer Durchführungsform;
- Figur 2: eine schematische Darstellung eines Messprinzips zum Ermitteln einer erforderlichen Kraft zum Bewirken des Blanching-Effekts auf der Haut eines Patienten;
- Figuren 3a, 3b: jeweils eine schematische Darstellung eines Farbmusterrings und eines zum Erfassen eines Farbtons eingerichteten elektronischen Messgeräts;
- Figur 4: eine schematische Darstellung eines Silikonkörpers gemäß einer Ausführungsform; und
- Figur 5: eine schematische Querschnitts-Darstellung einer Prothese umfassend den Silikonkörper aus Figur 4 gemäß einer Ausführungsform.

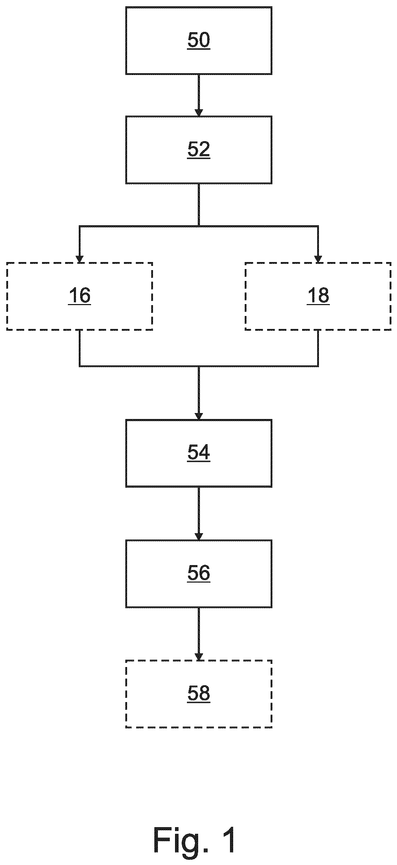

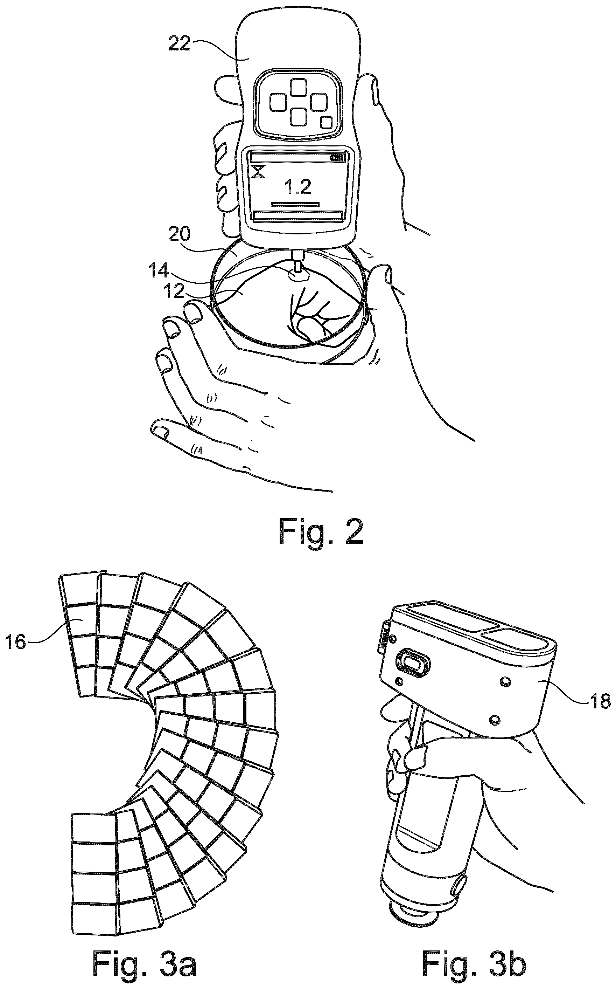

Figur 1 zeigt eine schematische Darstellung eines Verfahrens zum Herstellen eines Silikonkörpers 10 gemäß einer Durchführungsform. Das in Figur 1 dargestellte Verfahren wird im Hinblick auf die Figuren 2 bis 5 näher anhand eines herzustellenden Silikonkörpers 10 und einer den Silikonkörper 10 umfassenden Prothese 100 (oder einen Überzug dafür) für einen Finger als zu ersetzendes Körperteil erläutert, ohne darauf beschränkt zu sein. Vielmehr soll lediglich die zu Grunde liegende erfinderische Idee veranschaulicht werden. Der hergestellte Silikonkörper 10 und die Prothese 100 beziehungsweise der Überzug dafür können auch analog für andere zu ersetzende Körperteile herangezogen werden, wie beispielsweise für eine Hand, einen Arm, ein Bein, ein Fuß, ein Ohr und dergleichen. Die vorliegende Erfindung erlaubt es, die individuelle Naturfarbe der Haut eines Patienten 12 originalgetreu durch den Silikonkörper 10, wiederzugeben und damit eine besonders lebensechte Prothese 100 oder einen besonders lebensechten Überzug für eine Prothese 100 zu erreichen.

Der Kern des erfindungsgemäßen Verfahrens liegt in der Erkenntnis, dass der Farbton der blanchierten Haut entscheidend ist, um die individuelle Naturfarbe der Haut eines Patienten 12 mit der Prothese 100 wiedergeben zu können. Dies ist insbesondere erforderlich, da sich der Farbton der blanchierten Haut von Patient 12 zu Patient 12 (zumindest in Nuancen) unterscheidet. Überraschenderweise wird die originalgetreue Farbwiedergabe der Haut des Patienten 12 bei der Prothese 100 oder des Überzugs für eine Prothese 100 vergleichsweise einfach durch die Berücksichtigung des individuellen Farbtons der blanchierten Haut des Patienten 12 bei der Farbgebung des Silikonkörpers 10, der für die (spätere) Prothese 100 oder den Überzug für eine Prothese 100 des Patienten 12 herangezogen wird, ermöglicht. Folglich wird erfindungsgemäß nicht nur der Aufwand für eine möglichst originalgetreue Farbwiedergabe reduziert, sondern auch die Übereinstimmung der Farben grundsätzlich verbessert, wodurch die Lebensechtheit der (späteren) Prothese 100 oder des Überzugs für eine Prothese 100 verbessert wird.

In dem in Figur 1 gezeigten Verfahren zur Herstellung eines Silikonkörpers 10 wird in einem ersten Verfahrensschritt 50 ein Hautbereich eines Patienten 12 mit Kraft zum Blanchieren der Haut beaufschlagt. Das Blanchieren der Haut des Patienten 12 wird beispielhaft in Bezugnahme auf Figur 2 beschrieben.

In einem zweiten Verfahrensschritt 52 wird ein Farbton des blanchierten Hautbereichs 14 erfasst. Dieser Farbton wird bevorzugt unter Verwendung eines Farbmusterrings 16 (siehe Figur 3a) und/oder eines zum Erfassen eines Farbtons eingerichteten elektronischen Messgeräts 18 (siehe Figur 3b) erfasst. Bevorzugt wird der erfasste Farbton des blanchierten Hautbereichs 14 in einer numerischen Form beschrieben.

Gemäß einem dritten Verfahrensschritt 54 wird ein Silikon gemäß dem erfassten Farbton des blanchierten Hautbereichs 14 eingefärbt. Bevorzugt werden Farbpigmente in das Silikon eingeknetet oder in eine Silikon-haltige Lösung eingemischt. Das Silikon ist hierzu bevorzugt noch nicht ausvulkanisiert.

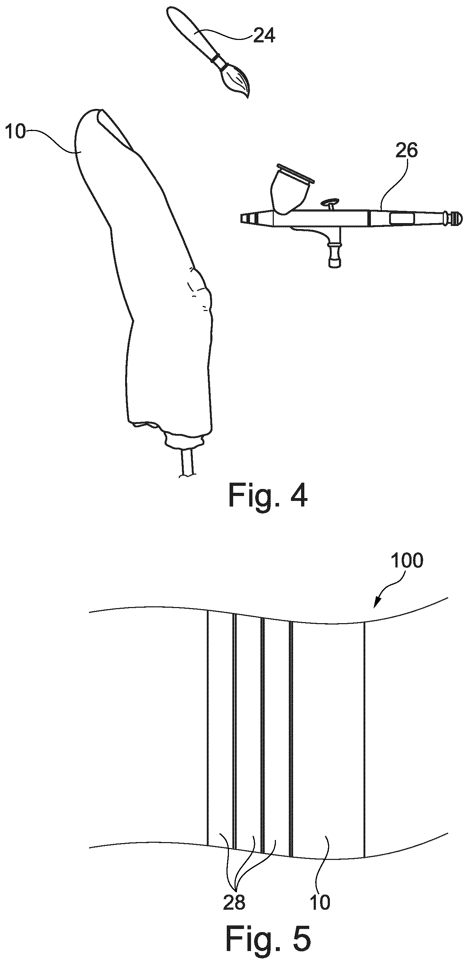

Ferner ist vorgesehen, dass ein Silikonkörper 10 basierend auf dem eingefärbten Silikon hergestellt wird (vierter Verfahrensschritt 56). Ein beispielhaft hergestellter Silikonkörper 10 ist in Figur 4 dargestellt. Bevorzugt wird das eingefärbte Silikon in eine gewünschte Form gebracht, beispielsweise in Form eines zu ersetzenden Fingers, und getempert. Bevorzugt wird der ausgeformte Silikonkörper 10 entsprechend den Herstellerangaben des gewählten Silikons in einem Ofen getempert, um den Vulkanisierungsprozess des Silikons zu bewirken. Beispielsweise wird der Silikonkörper bei 90 °C für zwei Stunden getempert. Durch den Vulkanisierungsprozess erhält das Silikon die zur späteren Verwendung gewünschten mechanischen Eigenschaften.

In einem optionalen fünften Verfahrensschritt 58 wird ein oder eine Vielzahl von Farbschichten 28 auf den hergestellten Silikonkörper 10 aufgetragen. Durch das Auftragen von Farbschichten 28 auf den eingefärbten Silikonkörper 10 kann dieser in lebensechter Farbgestaltung realisiert werden.

Figur 2 zeigt eine schematische Darstellung eines Messprinzips zum Ermitteln einer erforderlichen Kraft zum Bewirken des Blanching-Effekts auf der Haut eines Patienten 12. Dargestellt ist die Hand eines Patienten 12, eine durchsichtige Kunststoffplatte 20 und ein Kraftmessgerät 22 . Die durchsichtige Kunststoffplatte 20, beispielsweise in einer Ausformung wie eine Petrischale, wird auf die Haut des Patienten 12 an die entsprechende zu messende Hautstelle gelegt. Mit dem Kraftmessgerät 22 wird ein Druck beziehungsweise eine Kraft auf die Kunststoffplatte 20 ausgeübt, die sich auf die Haut überträgt. Bei einer ausreichenden Kraft tritt eine Blanchierung der Haut auf. Es kann dann also ein blanchierter Hautbereich 14 beobachtet werden. In dem in Figur 2 gezeigten Beispiel wird die Blanchierung der Haut auf der Außenseite der Hand am Knöchel vorgenommen und die dafür erforderliche Kraft mit dem Kraftmessgerät 22 ermittelt. Als Kraftmessgerät 22 wurde beispielsweise das digitale Messgerät Sauter FC 1K verwendet.

In Tabelle 1 sind ermittelte Kraftwerte jeweils für eine Hand mit dünner Haut, für eine Hand mit dicker Haut und für einen Fuß aufgeführt. Die Versuche haben gezeigt, dass je nach Beschaffenheit der Haut des Patienten 12 bereits ab 0,8 N erste Farbtonveränderungen durch den Blanching-Effekt zu verzeichnen sind. Je mehr Kraft aufgewendet wird, desto heller wird die Farbtonveränderung. Jedoch wurde ebenfalls festgestellt, dass ab einer gewissen Kraftschwelle keine weitere Farbtonveränderungen der Haut durch den Blanching-Effekt bewirkt werden können. Mit anderen Worten besteht oberhalb dieser Kraftschwelle ein Bereich mit einem konstanten Farbton des blanchierten Hautbereichs 14. Diese Kraftschwelle hängt im Einzelnen vor allem davon ab, ob eine Innen- oder Außenseite eines Körperteils gemessen wird, ob eine dicke oder dünne Haut der Messung zugrunde liegt. Ferner hängt die Kraftschwelle von dem jeweils betrachteten Körperteil ab.

**Tabelle 1: Ermittelte Kraftschwellen zum Bewirken des Blanching-Effekts auf Haut**

| | **Ermittelte Kraftschwelle für anfängliche Farbtonveränderung** | **Ermittelte Kraftschwelle für konstanten Farbton** |
|---|---|---|
| **Dünne Haut Handaußenseite** | Ab 0,8 N | Ab 10-12 N |
| **Dünne Haut Handinnenseite** | Ab 1N | Ab 15-17 N |
| **Dicke Haut Handaußenseite** | Ab 0,8 N | Ab 11-13 N |
| **Dicke Haut Handinnenseite** | Ab 1 N | Ab 16-18 N |
| **Fußrücken** | Ab 1 N | Ab 15-17 N |
| **Fußsohle** | Ab 1 N | Ab 17-19 N |

Die Figuren 3a und 3b zeigen jeweils eine schematische Darstellung eines Farbmusterrings 16 und eines zum Erfassen eines Farbtons eingerichteten elektronischen Messgeräts 18. Der Farbmusterring 16 umfasst eine Vielzahl von verschiedenen Farbmustern mit definierten Farbtönen. In dem gezeigten Beispiel sind 15 Farbmuster mit jeweils vier Abstufungen dargestellt, ohne jedoch hierauf beschränkt zu sein. Die Farbmuster können unmittelbar neben den blanchierten Hautbereich 14 gelegt werden, um eine Farbübereinstimmung des blanchierten Hautbereichs 14 mit einem der Farbmuster des Farbmusterrings 16 zu ermitteln. Dadurch, dass jedes Farbmuster einen definierten Farbton umfasst, ist das Erfassen des Farbtons des blanchierten Hautbereichs 14 auf diese Weise besonders einfach möglich.

Das in Figur 3b gezeigte zum Erfassen eines Farbtons eingerichtete elektronische Messgerät 18 ist bevorzugt ein Spektrometer oder ein Chromameter. Ein Spektrometer erlaubt es, die Intensität von Licht in Abhängigkeit der Wellenlänge zu erfassen. Mit anderen Worten kann mit einem Spektrometer ein Farbspektrum mit physikalischen Parametern erfasst und ausgegeben werden. Ein Chromameter ist dazu eingerichtet, Farbwerte in einem standardisierten Farbraum - typischerweise CIELAB (L*a*b*) - zu messen und diese als numerische Werte auszugeben. Die Verwendung des elektronischen Messgeräts 18 bietet eine objektive, reproduzierbare und einfache Art und Weise, einen Farbton des blanchierten Hautbereichs zu erfassen.

Bevorzugt wird die beaufschlagte Kraft zum Blanchieren der Haut direkt mit dem Farbmusterring 16 und/oder dem elektronischen Messgerät 18 ausgeübt. Im Falle des Farbmusterrings 16 kann dieser beispielsweise auf die durchsichtige Kunststoffplatte 20 gelegt werden und die Kunststoffplatte 20 auf die Haut gepresst werden, um den Blanching-Effekt hervorzurufen und den Farbton des blanchierten Hautbereichs anhand des Farbmusterrings 16 zu erfassen. Das elektronische Messgerät 18 umfasst bevorzugt einen lichtdurchlässigen (Glas-)Vorsatz zum Abtasten der Haut, der es ermöglicht, die Haut mit Kraft zu beaufschlagen und währenddessen den Farbton des blanchierten Hautbereichs 14 zu messen. Insofern ist eine besonders einfache und schnelle Art und Weise zum Erfassen des Farbtons des blanchierten Hautbereichs 14 gegeben. Bei einem beispielhaften Durchmesser des Aufsatzes des elektronischen Messgeräts 18 von 8 mm ergibt sich bei einer beaufschlagten Kraft von 1 N ein Druck auf die Haut von etwa 19,9 kPa.

Figur 4 zeigt eine schematische Darstellung eines Silikonkörpers 10 gemäß einer Ausführungsform. Der Silikonkörper 10 ist insbesondere durch das in Figur 1 gezeigte Verfahren hergestellt. Das heißt, der Silikonkörper 10 ist gemäß dem erfassten Farbton des blanchierten Hautbereichs 14 des Patienten 12 eingefärbt. Insofern ist der Silikonkörper 10 weißlich gelb bis elfenbeinfarben. Um die originalgetreue Farbwiedergarbe der Haut des Patienten 12 zu erreichen, wird eine oder eine Vielzahl von Farbschichten 28 auf den Silikonkörper 10 aufgetragen. Beispielhaft sind hierzu ein Pinsel 24 und eine Airbrushpistole 26 in Figur 4 gezeigt, die Beispielsystem zum Auftragen von Farbschichten 28 auf den Silikonkörper 10 darstellen und eine lebensechte, farbliche Bemalung des Silikonkörpers 10 ermöglichen.

Der hergestellte Silikonkörper 10 ist bevorzugt für eine Prothese 100 oder einen Überzug für eine Prothese 100 geeignet. In dem in Figur 4 gezeigten Beispiel ist der Silikonkörper 10 als ein Zeigefinger ausgeformt, ohne auf diese Form beschränkt zu sein. Es ist verständlich, dass eine Vielzahl von unterschiedlichen Anwendungsbereiche für Prothesen bestehen. Beispielhaft seien an dieser Stelle Arm-, Hand-, Bein-, Fuß, Ohrprothesen und ähnliche Extremitäten erwähnt.

Figur 5 zeigt schematische Querschnitts-Darstellung einer Prothese 100 umfassend den Silikonkörper 10 aus Figur 4 gemäß einer Ausführungsform. Neben dem weißlich gelb bis elfenbeinfarbenen Silikonkörper 10 sind eine Vielzahl von Farbschichten 28 auf dem Silikonkörper 10 aufgetragen. Beispielhaft sind drei Farbschichten 28 in Figur 5 angedeutet. Jedoch ist verständlich, dass die Anzahl sowie die Dicke der einzelnen Farbschichten 28 von der konkreten Hautfarbe des Patienten 12 abhängig sind. Bevorzugt besteht jede Farbschicht 28 aus einer Farbe. Die Farbe zwischen den Farbschichten 28 kann unterschiedlich oder auch zumindest teilweise oder vollständig gleich sein. Der Auftrag von Farbschichten 28 auf den Silikonkörper 10 erlaubt es, eine Farbgebung in Schichten von verschiedenen Farben, beispielsweise kaltes rot; warmes rot; gelb; blau; violett und dergleichen, aufzubauen, um eine lebensechte und patientenindividuelle Farbwirkung des Silikonkörpers 10 zu erzielen. Obgleich der Silikonkörper 10 für sich genommen mechanisch beziehungsweise funktionell bereits als eine Prothese 100 verstanden werden kann, soll der mit zumindest einer aufgetragenen Farbschicht 28 versehene Silikonkörper 10 hierin als Prothese 100 oder Überzug für eine Prothese 100 verstanden werden.

Das hierin beschriebene Herstellungsverfahren kann auch für die Herstellung von Silikonüberzügen für Prothesenfüße und Prothesen/Epithesen anderer Körperregionen angewendet werden.

### Bezugszeichenliste

- 10: Silikonkörper
- 12: Patient
- 14: blanchierter Hautbereich
- 16: Farbmusterring
- 18: elektronisches Messgerät
- 20: Kunststoffplatte
- 22: Kraftmessgerät
- 24: Pinsel
- 26: Airbrushpistole
- 28: Farbschicht

- 50: erster Verfahrensschritt - Beaufschlagen Hautbereich mit Kraft
- 52: zweiter Verfahrensschritt - Erfassen Farbton von blanchiertem Hautbereich
- 54: dritter Verfahrensschritt - Einfärben Silikon
- 56: vierter Verfahrensschritt - Herstellen Silikonkörper
- 58: optionaler fünfter Verfahrensschritt - Auftragen von Farbschichten

- 100: Prothese

## Patentansprüche

1. Verfahren zur Herstellung eines Silikonkörpers (10), umfassend die Schritte:
- Beaufschlagen (50) eines Hautbereichs eines Patienten (12) mit Kraft zum Blanchieren der Haut,
- Erfassen (52) eines Farbtons des blanchierten Hautbereichs (14),
- Einfärben (54) eines Silikons gemäß dem erfassten Farbton des blanchierten Hautbereichs (14) und
- Herstellen (56) eines Silikonkörpers (10) basierend auf dem eingefärbten Silikon.

2. Verfahren nach Anspruch 1, wobei die beaufschlagte Kraft mindestens 0,8 N oder mindestens 1 N beträgt.

3. Verfahren nach einem der vorangehenden Ansprüche, wobei die beaufschlagte Kraft in einem Kraftbereich liegt, in welchem eine weitere Erhöhung der beaufschlagten Kraft keine weitere Farbtonveränderung der blanchierten Haut erzeugt.

4. Verfahren nach einem der vorangehenden Ansprüche, wobei das Erfassen (52) des Farbtons des blanchierten Hautbereichs (14) unter Verwendung eines Farbmusterrings (16) und/oder eines zum Erfassen eines Farbtons eingerichteten elektronischen Messgeräts (18) erfolgt.

5. Verfahren nach dem vorangehenden Anspruch, wobei die beaufschlagte Kraft zum Blanchieren der Haut mit dem Farbmusterring (16) und/oder dem elektronischen Messgerät (18) ausgeübt wird.

6. Verfahren nach einem der Ansprüche 4 oder 5, wobei der Farbton des blanchierten Hautbereichs (14) unter Verwendung eines Abgleichs mit Farbtönen einer digitalen Farbbibliothek erfasst wird.

7. Verfahren nach einem der vorangehenden Ansprüche, wobei der Farbton des blanchierten Hautbereichs (14) am Handrücken und/oder am Fußrücken des Patienten (12) erfasst wird.

8. Verfahren nach einem der vorangehenden Ansprüche, wobei eine Vielzahl von Hautbereichen des Patienten (12) zum Blanchieren der Haut mit Kraft beaufschlagt wird, wobei die Farbtöne der Vielzahl von blanchierten Hautbereichen (14) erfasst werden und das Silikon basierend auf den erfassten Farbtönen der Vielzahl von blanchierten Hautbereichen (14) eingefärbt wird.

9. Verfahren nach einem der vorangehenden Ansprüche, ferner umfassend den Schritt:
- Auftragen (58) einer oder einer Vielzahl von Farbschichten (28) auf den hergestellten Silikonkörper (10).

10. Verfahren nach dem vorangehenden Anspruch, ferner umfassend den Schritt:
- Erfassen eines Farbtons des Hautbereichs (14) in unblanchierter Form, wobei das Auftragen einer oder einer Vielzahl von Farbschichten (28) auf den hergestellten Silikonkörper (10) basierend auf dem erfassten Farbton des Hautbereichs (14) in unblanchierter Form erfolgt.

11. Verfahren nach einem der vorangehenden Ansprüche, wobei der erfasste Farbton des blanchierten Hautbereichs (14) an einen Server mittels eines Kommunikationssignals übermittelt wird und das Einfärben des Silikons basierend auf dem auf dem Server hinterlegten erfassten Farbton des blanchierten Hautbereichs (14) erfolgt.

12. Verfahren nach einem der vorangehenden Ansprüche, ferner umfassend den Schritt:
- Lösen von Silikon in einem Lösungsmittel zum Erzeugen einer Silikon-haltigen Lösung, wobei die Silikon-haltige Lösung gemäß dem erfassten Farbton des blanchierten Hautbereichs (14) eingefärbt wird, und wobei das Herstellen des Silikonkörpers (10) basierend auf dem eingefärbten Silikon ferner die Schritte umfasst:
- Ausschwenken einer Negativform einer Prothese (100) oder einer Negativform eines Überzugs oder
- Übergießen und/oder Eintauchen einer Positivform der Prothese (100) oder einer Positivform des Überzugs,
wobei für das Ausschwenken oder das Übergießen und/oder das Eintauchen jeweils ein Fluid verwendet wird, welches die Silikon-haltige Lösung enthält oder daraus besteht.

13. Verfahren nach dem vorangehenden Anspruch, wobei das Silikon für den Silikonkörper (10) ein HTV-Silikon umfasst oder daraus besteht.

14. Silikonkörper (10) für eine Prothese (100) oder einen Überzug für eine Prothese (100) herstellbar nach einem Verfahren gemäß einem der vorangehenden Ansprüche.

15. Prothese (100) oder Überzug für eine Prothese (100) umfassend einen Silikonkörper (10) nach dem vorangehenden Anspruch.
